# EUROPEAN PATENT APPLICATION

(11) **EP 4 276 164 A1**
(43) Date of publication of application: **15.11.2023**
(21) Application number: 22172583.1
(22) Date of filing: 10.05.2022
(51) Int. Cl.: C12M 1/00

(54) **BIOREACTOR FOR CELL CULTURE WITH REUSABLE REACTOR VESSEL AND OPTICAL SPECTROSCOPY INTERFACE**

(71) Applicant: Sartorius Stedim Biotech GmbH, 37079 Göttingen (DE)
(72) Inventor: Höhse, Marek, 37079 Göttingen (DE); Grimm, Christian, 37079 Göttingen (DE); Reif, Oscar-Werner, 37079 Göttingen (DE)
(74) Representative: Prinz & Partner mbB

(57) **Abstract**

A bioreactor for cell cultivation comprises a reusable reactor vessel (16), preferably made of stainless steel, for receiving a fluid, and an optical spectroscopy interface (14) including an adapter (10) and a single-use optical spectroscopy insert (12). The reusable reactor vessel (16) has a wall (18) and a port formed in the wall (18). The adapter (10) is configured to be fixed to the port in a predefined position at the port. The single-use optical spectroscopy insert (12) is configured to be accommodated and fixed in the adapter (10), or the insert (12) is an integral part of the adapter (10).

## Description

The invention relates to a bioreactor for cell cultivation comprising a reusable reactor vessel and an optical spectroscopy interface.

Spectroscopy is increasingly used as a process analytical technology to monitor and control bioprocesses, especially in pharmaceutical manufacturing. With spectroscopic measurements one or more analytes can be monitored, even in real-time, without sample processing. However, implementation of spectroscopy systems has been hampered by their complexity.

From DE 10 2018 108 325 B4 a bioreactor comprising a container with a through-opening between the interior and the exterior of the container and a sensor receptacle that can hold a sensor is known. The sensor receptacle extends in the through-opening and has a window that seals the through-opening.

DE 10 2018 108 323 B4 shows a supporting device for an image capturing device on a bioreactor container. The supporting device includes a window and a holder. The window has a transparent element that is transparent to electromagnetic radiation. The holder is configured to hold the image capturing device. The supporting device is held at least partially in a through-opening of the container. The window seals the through-opening.

DE 10 2019 115 147 B4 relates to a biocompatible composite element for a bioreactor that includes an outer frame and an inner component. The outer frame is a polymeric material. The outer frame can be inseparably attached to a wall of the bioreactor. The inner component is a transparent material and secured in the outer component in an inseparable hermetically tight manner. The inner component is configured to enable a spectral process control.

None of these setups is specifically configured for the use of a single-use sensor, least of all for a dedicated single-use optical spectroscopy insert. Although the prior art solutions generally allow for an exchange of measuring devices for acquiring various physical, chemical or biological parameters during the production process, it has to be considered that such reusable measuring devices, especially fiber optical probes, used in reusable stainless steel vessels are neither identical nor compatible to single-use interfaces with respect to the optical setup. Accordingly, the use of a single-use interface results in a very complex transfer of the measurement results (spectra) and the mathematical evaluation model from one measurement system to the other.

In addition, reusable probes need to be steam sterilized (autoclaved) in-situ in the reactor vessel. This results in a reduced lifetime of the complex fiber optics and the whole probe. A frequent refurbishment is the consequence.

It is an object of the invention to overcome the above drawbacks and to provide a robust setup yielding reliable measurement results with reduced maintenance effort.

The above problem is solved by a bioreactor according to claim 1. Advantageous and expedient embodiments of the invention are apparent from the dependent claims.

The invention provides a bioreactor for cell cultivation, the bioreactor comprising a reusable reactor vessel, preferably made of stainless steel, for receiving a fluid, and an optical spectroscopy interface including an adapter and a single-use optical spectroscopy insert. The reusable reactor vessel has a wall and a port formed in the wall. The adapter is configured to be fixed to the port in a predefined position at the port. The single-use optical spectroscopy insert is configured to be accommodated and fixed in the adapter, or the insert is an integral part of the adapter.

The invention is based on the finding that most reusable reactor vessels have a port, e.g. a sampling port or an Ingold-Port, with a diameter sufficient to accommodate an optical spectroscopy interface including a single-use insert.

In general, the invention allows the use of hardware equipment designed for single-use applications to be used in reusable reactor vessels. In particular, the adapter of the optical spectroscopy interface, which can be a reusable or a single-use part, makes it possible to fit an optical spectroscopy insert made from a sterilizable plastic material and designed for a single use to a regular port of the reusable reactor vessel. A major advantage of this compatibility is that the optical probe head does not have to be autoclaved anymore because the single-use optical spectroscopy insert can be pre-sterilized before its implementation in the port of the reusable reactor vessel, and therefore the optics are not stressed by high temperatures and pressure of a sterilization process. Since the optical spectroscopy insert is only used once and is replaced by another insert for the next process, maintenance intervals of the bioreactor can be reduced.

In the context of the present invention, an optical spectroscopy insert includes the necessary structure for the fluid, such as a measurement chamber or a channel or the like, allowing optical measurements based on a spectroscopic technique, such as Raman, dynamic light scattering (DLS), multiangle light scattering (MALS), collimated transmission, NIR, MIR, UV, UV-Vis, 2D fluorescence etc., with the aid of a probe head.

Single-use components (disposables), such as the optical spectroscopy insert, are to be differentiated from reusable components which cannot simply be disposed and replaced after being used once, but must be cleaned, sterilized and tested again for each further use. It has to be understood that the person of ordinary skill in the art can well distinguish between dedicated single-use and multi-use/reusable equipment, especially in the field of biomanufacturing. It can always be clearly determined whether a component is a single-use or a reusable component due to its material and design. Such distinction is even possible for non-professionals in this technical field since laboratory equipment suppliers offer and market the single-use components with a clear marking/labelling, and the respective advantages and disadvantages compared to reusable components are discussed in detail. Thus, it should be clear that in the given context "single-use" does not mean something like "discardable".

Further, as the person of ordinary skill in the art is aware, dedicated single-use components are typically made from plastic materials, in particular sterilizable plastic materials which are suitable for autoclaving, sterilization by ionizing electromagnetic irradiation (gamma or beta radiation, X-rays) or chemical sterilization (ETO). This means that basic characteristics of such materials, like mechanical stability, toughness (opposite of brittleness) etc. and the dimensions of components made from such materials, are not significantly affected by a sterilization process. A cold-casted quartz component (used for optical purposes) also fulfills these criteria.

Preferably, the port of the reusable reactor vessel is formed according to an industry standard. For example, the port can be a standard sampling port or an Ingold port or a PG13.5 port. Such ports have defined shapes and a defined diameter. The adapter of the optical spectroscopy interface is designed to allow a single-use optical spectroscopy insert to be held in such a port.

In particular, the adapter can be configured to be reversibly connected directly to the (standard) port. As an alternative, the adapter can be directly or indirectly connected to a wall portion of the reusable reactor vessel surrounding the port.

Fixation in a certain orientation of the adapter holding the single-use optical spectroscopy insert can be aided by a fixation structure. The fixation structure can be part of the port, or it can be provided on the outer wall of the reusable reactor vessel. Preferably, the fixation structure allows fixation of the adapter on the reusable reactor vessel (only) in a defined position and orientation. This is of importance when a defined orientation of the single-use optical spectroscopy insert, which is accommodated in the adapter, is desired.

In this context, alignment means are expedient which define a given position and/or orientation of the single-use optical spectroscopy insert relative to the adapter and/or the reactor vessel. The alignment means may provide only a visible optical indication of the desired position and/or orientation of the insert. According to a more sophisticated approach, the alignment means may include a poka-yoke structure preventing an assembly in which the insert is in an undesired position and/or orientation.

Since a regular reusable reactor vessel itself usually does not include any means that can be used for a desired alignment of the insert, the alignment means, or at least a part of the alignment means, can be provided on the fixation structure.

The adapter of the optical spectroscopy interface preferably is a reusable part, e.g. made of stainless steel. However, the adapter can also be a single-use part made of a sterilizable plastic material as explained above.

In any event, the optical spectroscopy insert is a dedicated single-use component, preferably made mainly of a polymer material. Thus, the insert can be produced by a classic casting (hot or cold) or injection molding method or by an additive manufacturing method, such as 3D printing.

The adapter holding the single-use optical spectroscopy insert can be clamped towards the wall of the reactor vessel for fixation, e.g. by a screw connection or a bayonet mount. The resulting contact pressure can be exerted on a seal, preferably a gasket, which prevents leakage of fluid contained in the reactor vessel. In particular, the adapter can include a flange which interacts with a contact surface of the port, and the seal can effectively be provided between these surfaces.

Further, in order to prevent leakage between the adapter and the single-use optical spectroscopy insert, a seal should be provided between these components.

The single-use optical spectroscopy insert is preferably mounted from the inside of the reactor vessel. This ensures that the internal pressure of the fluid contained in the reactor vessel can be used to support the sealing effect and/or fixation of the insert in the adapter.

To allow for spectroscopic measurements, the single-use optical spectroscopy insert includes at least one spectroscopy window which is sufficiently transparent in the wavelength range of the radiation used for the measurements. To ensure that the measurements are not only representative for the portion of the fluid near the port, the fluid should move and pass the spectroscopy window. To this end, the insert includes a flow control structure, preferably including a slit or a channel, guiding fluid contained in the reactor vessel to pass through a flow passage along the spectroscopy window.

In order to provide the necessary flow of the fluid, a mixing means should be provided in the reusable reactor vessel.

According to a preferred embodiment of the invention, in the assembled state and use position of the bioreactor, the passage, through which the fluid flows and passes the spectroscopy window, defines a flow direction with an angle between 10° and 80°, preferably between 30° and 50°, relative to a horizontal plane. Such an orientation of the flow passage is particularly expedient to prevent air bubbles from being trapped (especially during filling) so that an ideal inflow is ensured.

According to a simple setup, the spectroscopy window is oriented in a plane that is not parallel, preferably orthogonal, to the adjoining portion of the wall of the reactor vessel. In this case, the optical path of the radiation used for the measurements does not have to be significantly changed in a probe head attached to the spectroscopy window. However, if the optical path is changed accordingly in the probe head, other orientations of the spectroscopy window are possible, especially an orientation parallel to the adjoining wall portion of the reactor vessel.

For spectroscopic measurements in transmission mode the single-use optical spectroscopy insert includes a second spectroscopy window opposite the first spectroscopy window. The flow passage then extends between the first and second spectroscopy windows.

For spectroscopic measurements in reflection mode the single-use optical spectroscopy insert includes a reflecting surface opposite the first spectroscopy window. Accordingly, the flow passage extends between the first spectroscopy window and the reflective surface. Depending on the specific type of the reflective measurements, the reflecting surface provides specular reflection or diffuse reflection.

According to an advanced embodiment of the invention the single-use optical spectroscopy insert includes a first spectroscopy pinhole on the side of the first spectroscopy window and/or a second pinhole on the side of the second spectroscopy window. Such pinholes can be used for so-called collimated transmission measurements.

In view of advantageous manufacturing techniques, the whole single-use optical spectroscopy insert can be made from a transparent material suitable for optical measurements based on a spectroscopic technique. Suitable materials include glass, quartz, PMMA, for example. In this case the spectroscopy window(s) is/are designed as a special integral part(s) of the entire insert. Such a "one piece made from a single material" insert can be efficiently manufactured by injection molding if the material is a transparent plastic such as PMMA, or by bonding or cold casting or additive manufacturing methods.

Preferably, the single-use optical spectroscopy insert is already adapted to be connected to a probe head so that no additional mounting parts are necessary.

Furthermore, connecting and/or alignment means can be provided on the insert and on the probe head for connecting and/or aligning the probe head relative to the single-use optical spectroscopy insert in a predefined position and/or orientation. The connecting and/or alignment means can include a hook structure engaging a rim or holes, or a thread connection or a bayonet mount, for example.

Further features and advantages of the invention will become apparent from the following description and from the accompanying drawings to which reference is made. In the drawings:
- Figure 1 shows a perspective front view of an adapter;
- Figure 2 shows a perspective rear view of the adapter;
- Figure 3 shows a perspective front view of a single-use optical spectroscopy insert;
- Figure 4 shows a perspective rear view of the single-use optical spectroscopy insert;
- Figure 5 shows a perspective front view of an optical spectroscopic interface formed by the adapter and the single-use optical spectroscopy insert, and a fixation structure with alignment means at a port of a bioreactor;
- Figure 6 shows a front view of a bioreactor according to the invention with an optical spectroscopic interface and a probe head;
- Figure 7 shows a cross section view along line A-A in Figure 6; and
- Figure 8 shows a front view of a port with an aligned optical spectroscopic interface;
- Figure 9 shows a detail of a cross section view along line C-C in Figure 8;
- Figure 10 shows a detail of a cross section view along line D-D in Figure 8; and
- Figure 11 shows a detail of a cross section view along line B-B in Figure 6.

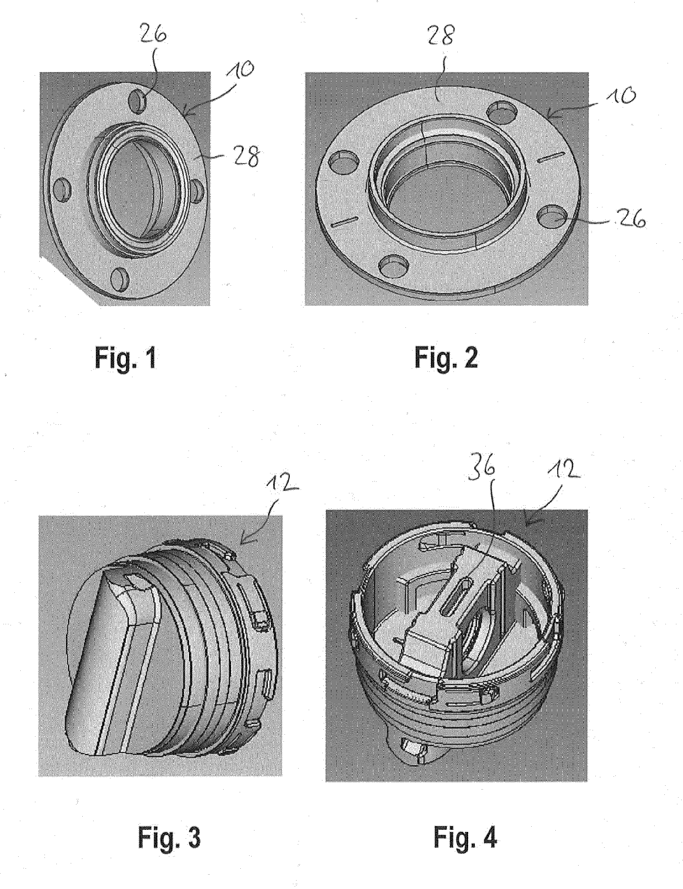

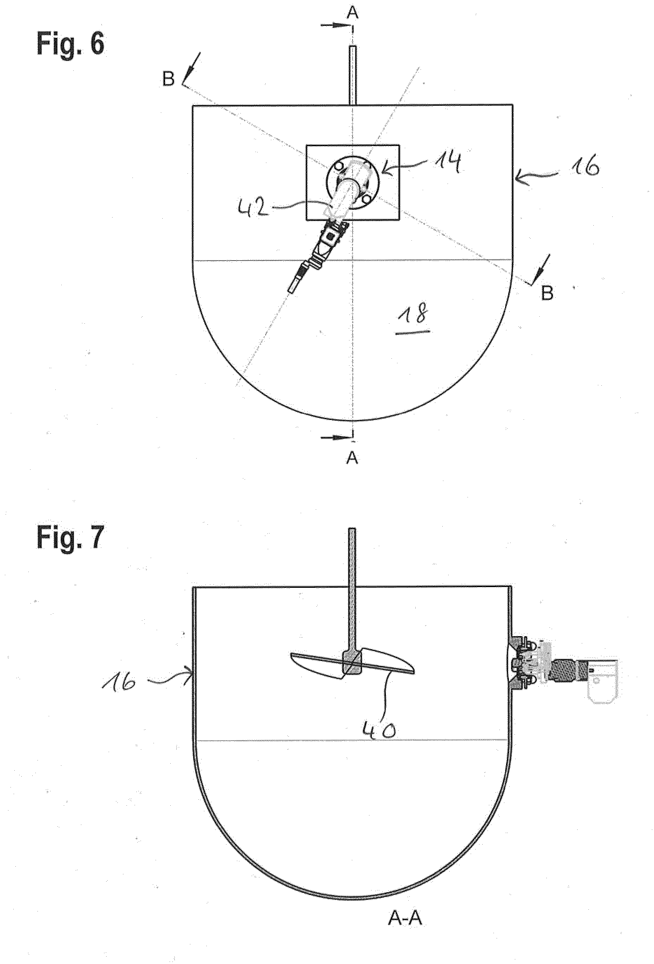

Figures 1 and 2 show an adapter 10, and Figures 3 and 4 show a single-use optical spectroscopy insert 12, which together form an optical spectroscopy interface 14. The optical spectroscopy interface 14 is intended for use with a bioreactor (see Figure 6) having a reusable reactor vessel 16 which is typically made of stainless steel.

The reactor vessel 16 includes a standard port provided in a wall 18 of the reactor vessel 16. The standard port is formed according to an industry standard and can be a standard sampling port or an Ingold port or a PG13.5 port, for example.

The adapter 10 is a reusable part, preferably also made of stainless steel, and is adapted to match the standard port of the reusable reactor vessel 16. The adapter 10 is a separate part which can be reversibly connected to the port, preferably via a mimic enabling a fixation of the adapter 10 directly to a matching mimic of the standard port. For example, a reversible fixation can be achieved by a threaded connection.

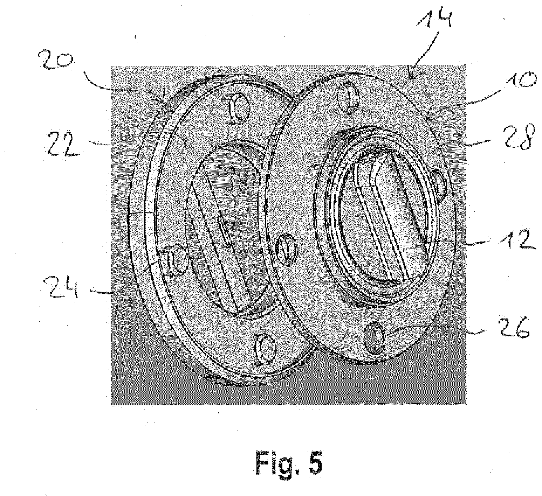

However, in the embodiment shown in the Figures, the adapter 10 is configured to be indirectly connected to a wall portion of the reactor vessel 16 surrounding the standard port. In particular, the adapter 10 is fixed to the wall portion via a fixation structure 20 which is irreversibly mounted to the wall 18 of the reactor vessel 16 (see Figure 5). The fixation structure 20 includes an annular sealing surface 22 with a number of threaded holes 24. The threaded holes 24 correspond to fixation holes 26 formed in a flange 28 of the adapter 10. The adapter 10 is attached to the fixation structure 20 by screws (not shown) engaging through the fixation holes 26 and threaded into the threaded holes 24 of the fixation structure 20. Thus, a contact pressure is created between a rear surface of the flange 28 of the adapter 10 and the opposing sealing surface 22 of the fixation structure 20. The arrangement of the holes 24 and 26 can be used to allow only one orientation (or only a limited number of orientations) of the adapter 10 relative to the wall 18 of the reactor vessel 18.

As an alternative, the contact pressure between a surface of the adapter 10 and a surface of the fixation structure 20 can also be created by a bayonet mount. In this case, the adapter 10 and the fixation structure 20 include matching slots and protrusions.

The actual sealing effect is established by a seal (not shown), especially a gasket, clamped between the two surfaces. The seal can be a separate part, or it is formed directly on one of the surfaces.

In any event, the adapter 10 is mounted in a predefined orientation relative to the reactor vessel 16.

The other essential part of the optical spectroscopy interface 14 is the single-use optical spectroscopy insert 12 mainly made from sterilizable plastic material, such as a polymer material, and is preferably manufactured by an additive manufacturing method (e.g. 3D printing), or by casting (hot or cold) or injection molding. The single-use optical spectroscopy insert 12 can also be made from another material, such as a quartz block, by a cold casting technique, or from several of such single-use materials.

The insert 12 is accommodated and fixed in the adapter 10. The fixation can be realized via screws or a clip connection or, as shown in Figures 1 to 4, a bayonet mount, creating a contact pressure. Sealing is provided via a sealing surface or via a circumferential seal. As an alternative, especially when the adapter 10 is made of a plastic material, the insert 12 can also be glued to the adapter 10.

The insert 12 is mounted to the adapter 10 from the inside of the reactor vessel 16, so that the internal pressure of the fluid contained in the reactor vessel 16 provides an (additional) contact pressure, causing the insert 12 to be pressed against the adapter 10. This also contributes to the sealing effect. However, other designs are possible, enabling the insert 12 to be mounted more easily from the outside of the reactor vessel 16.

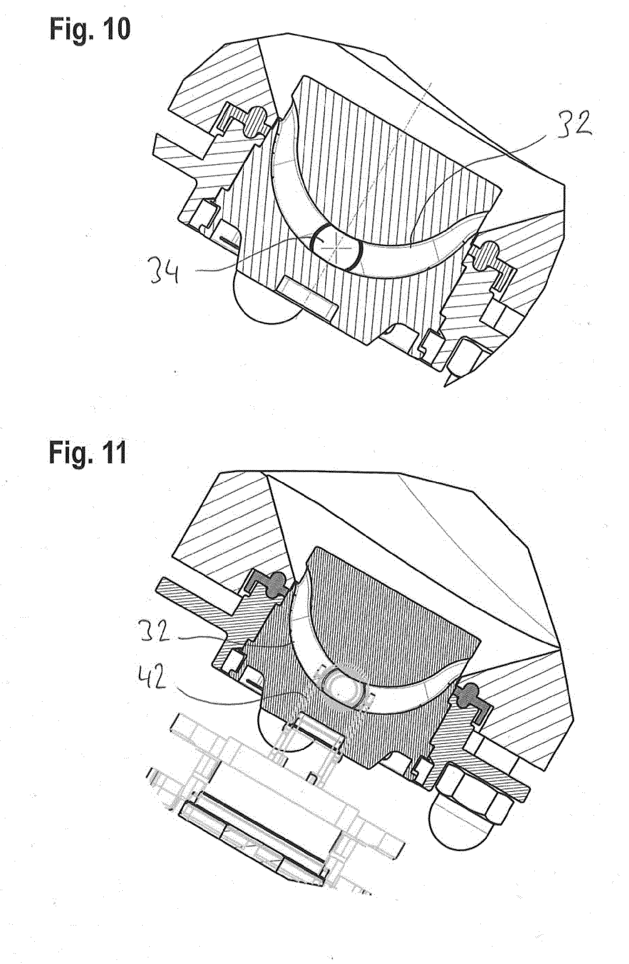

The following description also makes reference to Figures 6 to 11.

The single-use optical spectroscopy insert 12 is in direct contact with the fluid contained in the reactor vessel 16. The insert 12 includes at least one spectroscopy window 30 and a flow control structure 32 including a slit or a channel, guiding the fluid contained in the reusable reactor vessel 16 to pass through a flow passage along the first spectroscopy window 30.

In the assembled state and use position of the bioreactor, the first spectroscopy window 30 is oriented in a plane that is parallel to the adjoining portion of the wall 18 of the reactor vessel 16.

On the other side of the slit or channel, opposite the first spectroscopy window 30, a parallel second spectroscopy window 34 can be provided.

The spectroscopy windows 34 can consist of optical glass, or a transparent crystal such as MgF₂, CaF₂, sapphire, quartz, or a transparent plastic, or a semiconductor that is transparent for IR radiation such as Si, Ge, etc.

Each spectroscopy window 30, 34 should be at least 50 % transparent for at least parts of the wavelength range used for the respective measurement technique. For example a window could be transparent in a wavelength range between 190 and 2500 nm. If MIR spectroscopy is to be used, the material needs to be sufficiently transparent in the corresponding wavelength between 3000 and 8000 nm.

The second window can also be used for excitation/emission spectroscopy (fluorescence), or two different measuring can be performed in the same observation volume simultaneously or alternately (e.g. NIR diffuse reflectance from one side, Raman from the other side).

One or both of the spectroscopy windows 30, 34 can consist of an ATR crystal to allow measurement in the evanescent field (e.g. MIR).

In addition to or instead of the second spectroscopy window 34 a reflecting surface can be provided which is preferably oriented parallel with respect to the first spectroscopy window 30. The reflecting surface can be configured to provide specular reflection or diffuse reflection.

The flow passage formed by the slit or channel between the first and second spectroscopy windows 30, 34, or between the first spectroscopy window 30 and the reflecting surface, has a width in the range of 0.1 to 10 mm, preferably around 1 mm. However, for some applications a design with a width lower than 0.1 mm or greater than 10 mm can be chosen (e.g. for cell-free process steps, or for trace detection, e.g. DNA in high protein concentration, respectively).

As can be concluded from Figures 5, 6 and 8, the flow passage is tilted with respect to a horizontal plane, i.e. the flow passage extends in an angle between 10° and 80°, preferably between 30° and 50°.

The desired orientation of the insert 12 relative to the adapter 10 and/or relative to the reactor vessel 16 is defined by special alignment means. Here, the alignment means include a first alignment contour 36 on the insert 12 and a matching second alignment contour 38 on the fixation structure 20, as can be seen in Figures 4 and 5, respectively. Preferably, the alignment contours 36, 38 interact with each other according to the poka-yoke principle in order to avoid an incorrect or undesired assembly. According to a simpler approach, the alignment means only visually indicate whether the insert 12 is in the desired orientation or not.

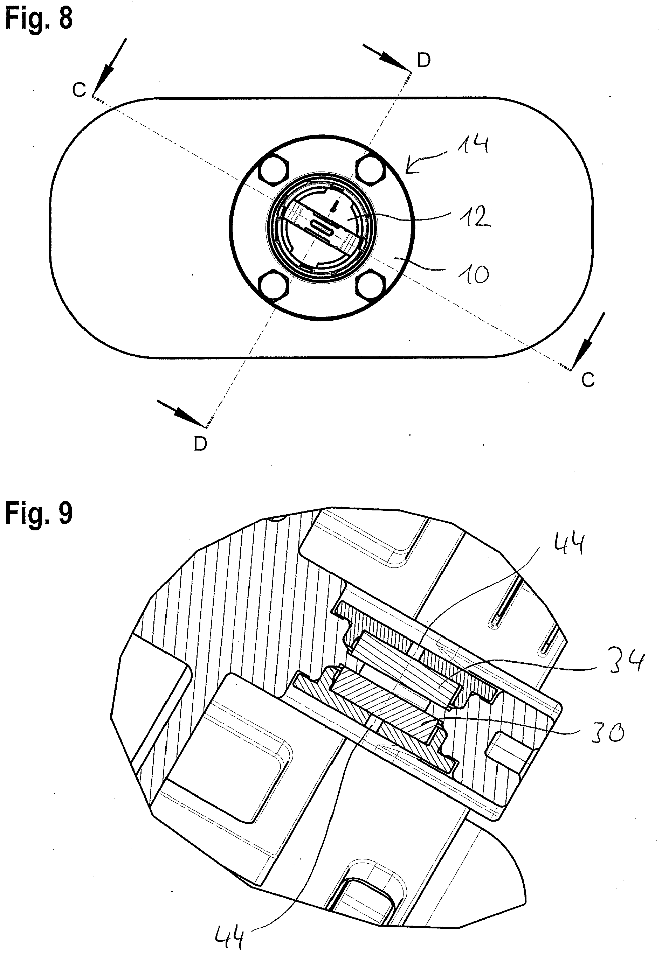

A flow of the fluid contained in the reactor vessel 16 through the flow passage, especially when the flow passage extends in an oblique upward direction (see the arrows indicating the flow direction in Figures 6 and 8), can be provided or supported by mixing means 40 arranged in the reusable reactor vessel 16, such as a stirrer or an impeller, which is mainly used to stir the fluid in the reactor vessel 16.

Depending on the actual setup (two spectroscopy windows 30, 34 or one window 30 and a reflecting surface), optical measurements in transmission mode, (specular or diffuse) reflection mode or transflection mode can be performed.

For performing the optical measurements, a probe head 42 (see Figures 6, 7 and 11), which can be coupled to one or more optical fibers, is reversibly connected to the single-use optical spectroscopy insert 12. The probe head 42 has a hook structure engaging behind a rim of the insert 12. Another kind of a clip connection or a screw connection or a bayonet mount is also possible to attach the probe head. If necessary, the correct positioning and/or orientation of the probe head 42 can be ensured by alignment means in a manner as described above in connection with the orientation of the insert 12 in the adapter 10.

Depending on the orientation of the optical path in the probe head, an orthogonal orientation of the spectroscopy window(s) 30, 34 and/or the reflecting surface relative to the the adjoining portion of the wall 18 of the reactor vessel 16 may be beneficial.

The spectroscopy window(s) 30, 34 of the single-use optical spectroscopy insert 12 can be shaped and oriented such that angular resolved measurements are possible (measurements at a plurality of different angles). For this, the respective window would need several surfaces formed at equal angles (such as in a half hexagon). An optical fiber input of a probe or a detector is positioned at each exit surface of the window.

Furthermore, the first spectroscopy window 30 (entrance window) can be configured to allow for measurements based on attenuated total reflectance (ATR). For this, trapezoidal crystals with two parallel optical surfaces can be used, between which multiple reflection can take place, and where one of the two sides (the shorter one) points to the sample. For MIR-ATR, diamond, silicon or germanium is used as material.

According to a variant, one or two pinholes 44 (see Figure 9) are provided next to the spectroscopy window(s) 30, 34, e.g. in the structure holding the windows 30,34, for so-called collimated transmission measurements. Each pinhole 44 is arranged on the outer side of the respective window directly before the entrance to an optical fiber or a detector, so that they do not come into contact with the fluid of the reactor vessel 16.

The adapter 10 and the single-use optical spectroscopy insert 12 can be individual parts as shown in the Figures. However, according to an alternative embodiment, the adapter 10 and the single-use optical spectroscopy insert 12 are made as a single piece from the same main material. In this case the whole optical spectroscopy interface 14 is a single-use component.

The optical spectroscopy interface 14 formed by the adapter 10 and the single-use optical spectroscopy insert 12 can be used in connection with any suitable fiber or fiber-free spectroscopy measurement system, such as Raman, dynamic light scattering (DLS), multiangle light scattering (MALS), collimated transmission, NIR, MIR, UV, UV-Vis, 2D fluorescence etc.

### List of Reference Signs

- 10: adapter
- 12: single-use optical spectroscopy insert
- 14: optical spectroscopic interface
- 16: reusable reactor vessel
- 18: wall
- 20: fixation structure
- 22: sealing surface
- 24: threaded holes
- 26: fixation holes
- 28: flange
- 30: first spectroscopy window
- 32: flow control structure
- 34: second spectroscopy window
- 36: first alignment contour
- 38: second alignment contour
- 40: mixing means
- 42: probe head
- 44: pinholes

## Claims

1. A bioreactor for cell cultivation, comprising a reusable reactor vessel (16), preferably made of stainless steel, for receiving a fluid, and an optical spectroscopy interface (14) including an adapter (10) and a single-use optical spectroscopy insert (12), the reusable reactor vessel (16) having a wall (18) and a port formed in the wall (18), the adapter (10) being configured to be fixed to the port in a predefined position at the port, and the single-use optical spectroscopy insert (12) being configured to be accommodated and fixed in the adapter (10) or being an integral part of the adapter (10).

2. The bioreactor according to claim 1, **characterized in that** the adapter (10) is configured to be reversibly connected either directly to the port, or directly or indirectly to a wall portion of the reusable reactor vessel (16) surrounding the port.

3. The bioreactor according to any of the preceding claims, **characterized by** a fixation structure (20) allowing fixation of the adapter (10) to the reusable reactor vessel (16) in a defined position and orientation.

4. The bioreactor according to any of the preceding claims, **characterized by** alignment means defining a given position and/or orientation of the single-use optical spectroscopy insert (12) relative to the adapter (10) and/or the reactor vessel (16).

5. The bioreactor according to any of the preceding claims, **characterized in that** the adapter (10) is clamped towards the wall (18) of the reactor vessel (16) and includes a seal, preferably a gasket, preventing leakage of fluid contained in the reactor vessel (16).

6. The bioreactor according to any of the preceding claims, **characterized by** a seal between the adapter (10) and the single-use optical spectroscopy insert (12).

7. The bioreactor according to any of the preceding claims, **characterized in that** the single-use optical spectroscopy insert (12) includes at least a first spectroscopy window and a flow control structure, preferably including a slit or a channel, guiding fluid contained in the reactor vessel (16) to pass through a flow passage along the first spectroscopy window.

8. The bioreactor according to claim 7, **characterized in that** a mixing means is provided in the reusable reactor vessel (16).

9. The bioreactor according to claim 7 or 8, **characterized in that**, in the assembled state and use position of the bioreactor, the flow passage defines an oblique flow direction with an angle between 10° and 80°, preferably between 30° and 50°, relative to a horizontal plane.

10. The bioreactor according to any of claims 7 to 9, **characterized in that**, in the assembled state and use position of the bioreactor, the first spectroscopy window is oriented in a plane that is not parallel, preferably orthogonal, to the adjoining portion of the wall (18) of the reactor vessel (16).

11. The bioreactor according to any of claims 7 to 10, **characterized in that** the single-use optical spectroscopy insert (12) includes a second spectroscopy window opposite the first spectroscopy window, the flow passage extending between the first and second spectroscopy windows.

12. The bioreactor according to any of claims 7 to 11, **characterized in that** the single-use optical spectroscopy insert (12) includes a reflecting surface opposite the first spectroscopy window, the flow passage extending between the first spectroscopy window and the reflective surface.

13. The bioreactor according to any of claims 7 to 12, **characterized in that** the single-use optical spectroscopy insert (12) includes a first spectroscopy pinhole on the side of the first spectroscopy window and/or a second pinhole on the side of the second spectroscopy window.

14. The bioreactor according to any of claims 7 to 13, **characterized in that** the whole single-use optical spectroscopy insert (12) is made from a transparent material suitable for optical measurements based on a spectroscopic technique.

15. The bioreactor according to any of the preceding claims, **characterized in that** the single-use optical spectroscopy insert (12) is adapted to be connected to a probe head, and connecting and/or alignment means are provided on the insert (12) and on the probe head for connecting and/or aligning the probe head relative to the single-use optical spectroscopy insert (12) in a predefined position and/or orientation.
